# EUROPEAN PATENT SPECIFICATION

(11) **EP 3 454 763 B1**
(45) Date of publication and mention of the grant of the patent: **07.12.2022**
(21) Application number: 17729025.1
(22) Date of filing: 11.05.2017
(51) Int. Cl.: A61B 17/12, A61N 1/05, A61N 1/36

(54) **NON-IMPLANT DEVICE FOR TEMPORARY OCCLUSION OF BLOOD VESSELS**
NICHTIMPLANTATVORRICHTUNG ZUR TEMPORÄREN OKKLUSION VON BLUTGEFÄSSEN
DISPOSITIF NON IMPLANTABLE POUR L'OCCLUSION TEMPORAIRE DE VAISSEAUX SANGUINS

(30) Priority: 13.05.2016 EP 16169535
(43) Date of publication of application: 20.03.2019
(73) Proprietor: Swiss VX Venentherapie und Forschung GmbH, 8834 Schindellegi Gem. Feusisberg (CH)
(72) Inventor: RAGG, Christof, 10789 Berlin (DE)
(74) Representative: CH Kilger Anwaltspartnerschaft mbB
(86) International application number: PCT/EP2017/061381
(87) International publication number: WO 2017/194698

(56) References cited:
- EP-A1- 2 656 869
- US-A1- 2008 312 679
- US-A1- 2010 106 178
- US-A1- 2011 046 543
- US-A1- 2012 143 301
- US-A1- 2016 051 263

## Description

### Field of the invention

The present invention is in the field of medical devices, in particular in the field of catheter devices. The present invention relates to a catheter assembly.

### Background

Human veins of the lower extremity are classified according to their relationship to the muscular fascia and are located in either the superficial or deep compartment. The venous system of the lower extremities includes the deep veins that lie beneath the muscular fascia and drain the lower extremity muscles; the superficial veins that are above the deep fascia and drain the cutaneous microcirculation; and the perforating veins that penetrate the muscular fascia and connect the superficial and deep veins. The venous system contains numerous one-way valves for directing blood flow back to the heart. When the valves of the veins fail to work properly, a series of disorders arise, such as venous insufficiency. This interferes with venous return and causes the blood to pool in the veins (Meissner, Seminars in Interventional Radiology 2005; 22 (3): 147-156). Besides the aesthetic issue, venous insufficiency leads to major complications due to the congestion and the poor circulation through the affected limb. The complications comprise: spider veins, varicose veins, phlebitis, blood clots and changes in the skin. The most serious disorder is a venous leg ulcer. Chronic venous disorders (CVD) are a collective term used to describe a long-standing condition involving impaired venous return in varying degrees of severity.

Varicose veins are the most common complications, characterized by the presence of tortuous, twisted or elongated veins. This can be due to existing (inherited) valve dysfunction or decreased vein elasticity (primary venous reflux) or valve damage from prior thrombotic events (secondary venous reflux). The result is pooling of blood in the veins, increased venous pressure and subsequent vein enlargement leading to varicosities (varicose veins). Symptoms typically affect the lower extremities and include: aching, swelling, throbbing, night cramps, restless legs, leg fatigue, itching and burning. Left untreated, venous reflux tends to be progressive, often leading to chronic venous insufficiency. A number of complications are associated with untreated venous reflux including superficial thrombophlebitis as well as rupture of varices and hemorrhage. For varicose veins, surgical removal of the target structure has been a widely-used therapy for decades. However, like all surgical treatments this may be accompanied by several, partially serious adverse effects, comprising damaging of adjacent arteries, nerves or lymphatic vessels, generation of wounds and cicatrices, wound infections or intolerance of the patient for narcotic drugs. Furthermore, the tissue damage going along with every surgery, in particular in junction regions like the groin or the poplitea, seems to induce the growth of new diseased veins. As an alternative to surgical removal, different ways of endovenous closure methods have been developed. The term endovenous means that therapy is performed by access through the venous system and within the diseased vein. The aim of these methods is the permanent closure of the treated vein or vein segment. The effect may be obtained by thermal treatment (e.g. by laser, radiofrequency, steam), or by injection of chemical agents (fluids, foams). Due to the use of catheters and probes, thermal treatment is restricted to relatively linear vessels while chemical agents, such as sclerosant compounds may also reach curved and tortuous segments or branched vein.

The objective of sclerotherapy is generally to destroy the endothelium of the target vessel by injecting an irritant solution (for example a detergent, osmotic solution, or a chemical irritant), ultimately resulting in the complete obliteration of the vessel. Known liquid sclerosant drugs are e.g. alcohols with detergent properties like polidocanol or sodium tetradecyl sulphate. In the eldest modality, the liquid sclerosant drug is injected directly into the vessels. Due to its high fluidity, the liquid sclerosant drug flows with the blood stream and quickly mixes with blood, soon reaching ineffective dilutions. Protein bindings additionally limit the effect of fluid sclerosant agents. In order to circumvent some drawbacks of the liquid sclerosant drugs, one usually makes a sclerosant foam by mixing the liquid sclerosant drug with a gas. The resulting sclerosant drug foam is injected into the target structure, e.g. the varicose vein. For foaming the sclerosant drug (e.g. Sodium Tetradecyl Sulfate or polidocanol) is mixed with sterile air or physiological gases (e.g. carbon dioxide, oxygen) in a syringe or by using mechanical pumps. Foaming increases the surface area of the drug. Due to its higher viscosity, the sclerosant drug foam is more efficient than the liquid sclerosant drug in causing sclerosis, thickening of the vessel wall and sealing off the blood flow (Dermatol. Surg. 2004, 30 (5): 718-22; Dermatol. Surg. 2003, 29 (12): 1170-1175). Catheter-injected sclerofoam is the only approved method offering therapy of a large vein (e.g. saphenous veins) and its side branches or connections to the deep vein system (so called perforator veins) from a single access point. The success of the treatment may depend on different parameters such as accurate injection in the target vessel, an adequate volume and concentration of sclerosant agent and a post-procedure compression. Moreover, the use of sclerotherapy, as opposed to the physical removal of the vein with stripping, raises the issue of recurrence due to recanalization.

The problem of inaccurate injection is not only related to liquid and/or foamed sclerosants, but also to gel-like sclerosants or glue. Manufacturers of vein gluing systems insist on a safety distance of several centimeters to the point intended to treat. However, in doing so, an untreated area remains that represents a source of later recurrence.

During the last years, some vessel blocking devices and methods have been developed, inter alia, to support sclerotherapy. These vessel blocking devices mainly refer to stent implants which permanently block at least part of a vessel and the blocked section of the vessel may be treated using, for example, ligation, heat and/or sclerosing or other suitable agents. However, today's stent implant technique is not yet satisfactory. Current implantable vessel devices present some drawbacks including the fracture and migration of the implant or parts of it in the circulatory system which may have serious consequences. The most common mechanisms of stent fracture are represented by metal fatigue due to mechanical forces and shearing stress. Particularly, the interaction between stent and vessel geometry during stent implantation is considered the most important factor in the pathophysiology of stent fracture. Moreover, stents change the vessel geometry, thus creating a new vessel angulation. This geometric distortion imposes a considerable mechanical force, increasing metal fatigue and finally the likelihood of stent fracture (Curr. Cardiol. Rev. 2014, 10(4): 349-354.). When used in veins close to the skin, external forces may lead to deformation, fracture or migration. Migration of venous implants may happen in spite of proper implantation due to the weak vein wall. If hooks are used to fix the implant, these may invade the vein wall and cause perforations. As a consequence, there is a concrete need in the art for new medical devices which provide a temporary occlusion of the blood vessels and which are usable in diverse segments of blood vessel.

Document US2016051263 A1 describes an endovascular clip comprising a proximal anchoring member and a distal self-expanding member. The proximal anchoring member and distal self-expanding member are configured to extend across opposite sides of a neck of an aneurysm. The distal self-expanding member may be joined to a connecting joint. The connecting joint may serve as a central pivot ring, around which the distal self-expanding member may be angled upwards and downward.

Document US2012143301 A1 describes an intravascular delivery device comprising a delivery wire having a proximal and a distal end and an interior lumen extending there between and wherein said distal end comprises a connection interface adapted to matingly interlock with a proximal end portion of a medical implantable device, wherein said delivery device comprises a locking unit arranged to secure said connection interface in a locking position in which said medical implant is pivotably locked before a controlled release.

Document US2011046543 A1 describes a method for collapsing a target vein in a patient. The method comprises providing an intravascular irritation element having a plurality of mechanical irritating objects, inserting the intravascular irritation element into a venous lumen of a target vein, and irritating the target vein by moving the plurality of mechanical irritating objects in contact with the inner surface thereof, thereby triggering a collapse of said target vein. A sclerosing agent may be released into the venous lumen.

### Summary of the invention

The present invention is defined by appended claim 1. Specific embodiments are set forth in the dependent claims.

### Brief Description of Drawings

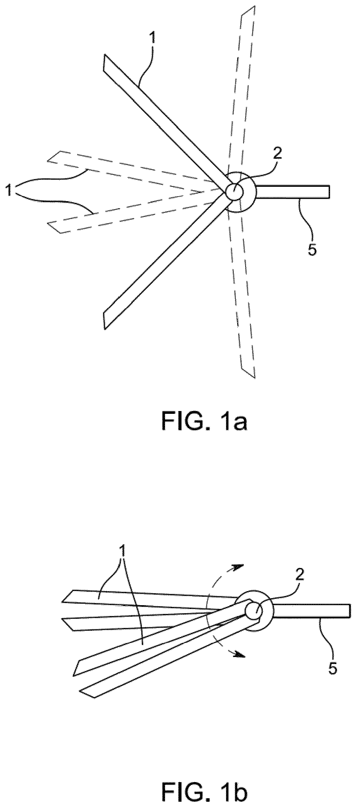

Figure 1 shows a side view of the expandable assembly (1), in expanded state (a) and retracted state (b). In the present exemplary figure, a pivotable joint (2) can be located at the basis of the expandable assembly or at a distance of 0,5 - 5 cm, preferably 1 - 3 cm from the basis of said expandable assembly and connects the expandable assembly to the steerable guide wire (5).

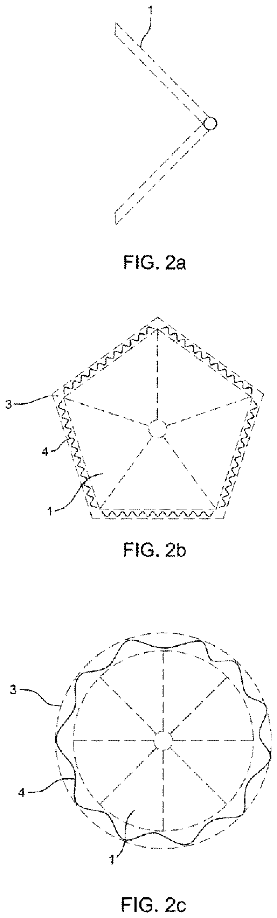

Figure 2 shows the expandable assembly (1) in the expanded state, side view section (a) and bottom view (b, c). The dashed-line indicates elements whose shape may change according to state of said expandable assembly (1). The expandable assembly (1) may comprise an adhesive or sealing component (4) suitable for achieving a temporary bond between the border of the expandable assembly (3) and the internal wall of the vessel intended to occlude. The border of said expandable assembly (3) may have a polygonal shape (b) or a circular shape (c).

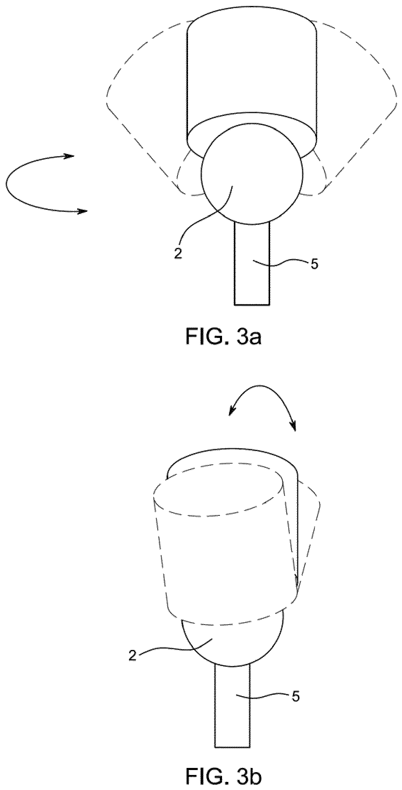

Figure 3 shows a side view of the pivotable joint (2) of the guide wire (5) allowing for deflecting of the expandable assembly (not shown) in two or three dimensions relative to said guide wire (5).

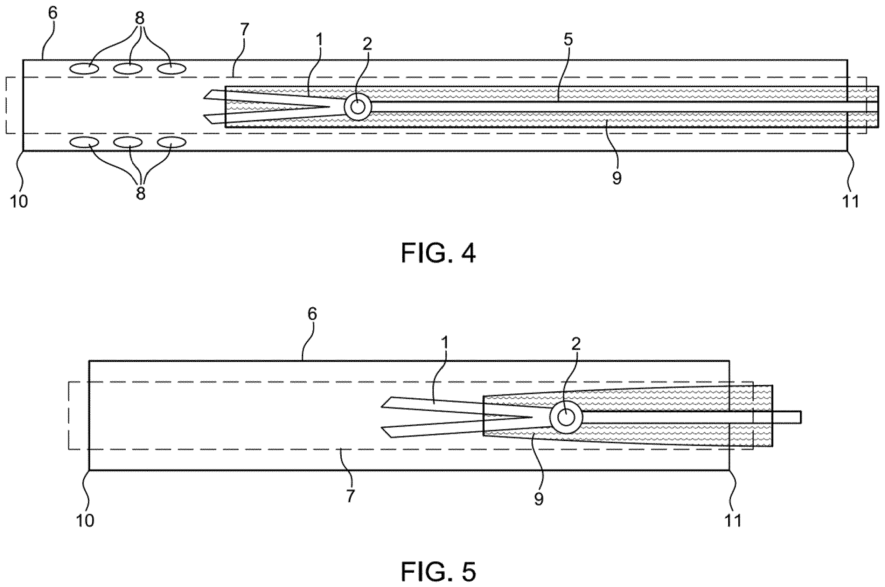

Figure 4 shows the catheter assembly according to the present invention, comprising a first tube (6) and a guide wire (5) located within said first tube (6), optionally wherein said guide wire (5) is located in a second tube (7) (dashed-line) positioned within said first tube (6). The first tube (6) may comprise a plurality of side holes (8) for dispensing sclerosant foam and/or aspirating fluids.
Figure 5 shows a lateral section of the catheter assembly comprising a sliding sleeve (9) which accommodates the expandable assembly (1) in a retracted state within the catheter assembly. The sliding sleeve (9) is comprised within the outer tube (6) of the catheter device.

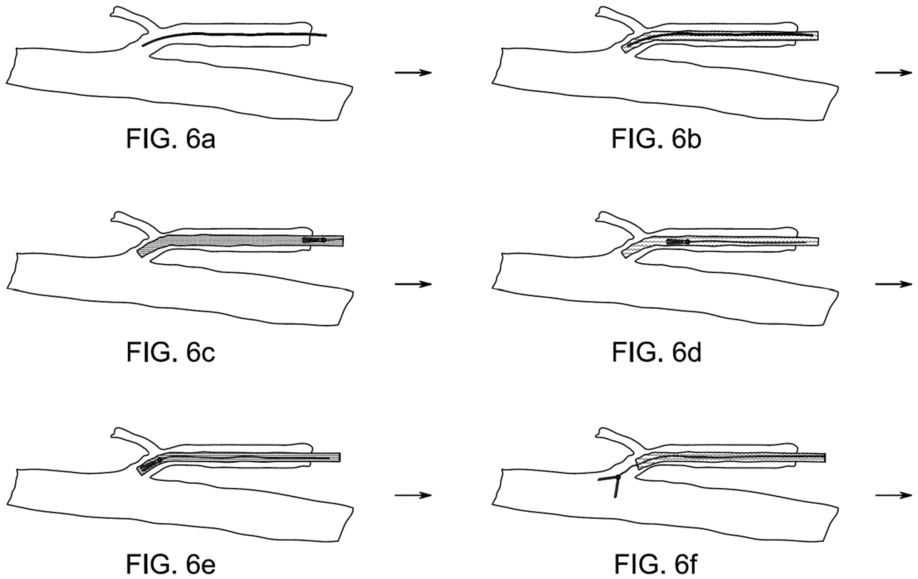

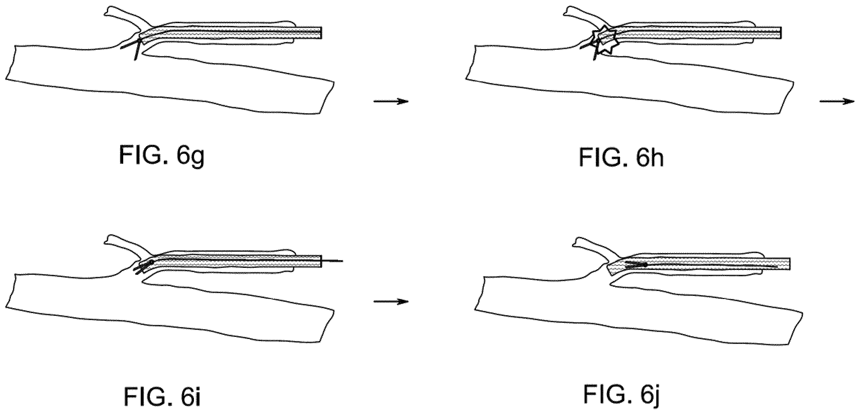

Figure 6 shows the use of a guide wire (5) according to the present disclosure for temporary blood vessel occlusion. The main steps may be resumed as follows: a) insertion of a standard guide wire; b) advancing the catheter to the desired position; c) removing the standard guide wire and loading of the expandable assembly and its guide wire into the catheter; d) advancing the guide wire within the catheter; e) positioning the catheter assembly over the location intended to occlude; f) withdrawing the catheter while the guide wire is kept in place, until the expandable assembly is released and expanded; g) retracting and positioning the expanded assembly; h) performing foam sclerotherapy; i) advancing the catheter to the location temporarily occluded to collect the expanded assembly; j) retracting the expanded assembly by pulling its guide wire to a position within the catheter.

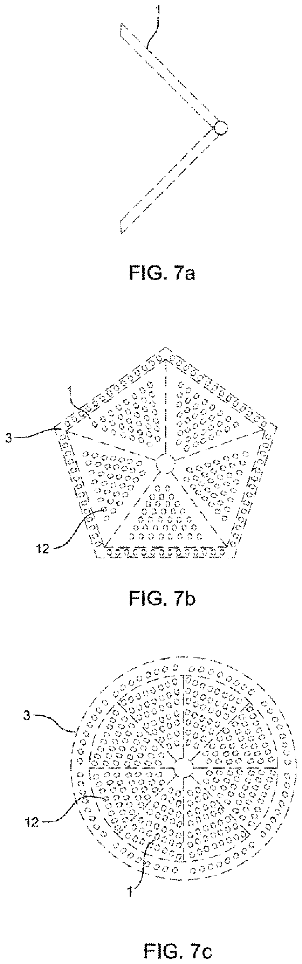

Figure 7 shows the expandable assembly (1) in the expanded state according to the third aspect of the present disclosure, side view section (a) and bottom view (b, c). The dashed-line indicates elements whose shape may change according to state of said expandable assembly (1). The expandable assembly (1) may comprise one or a plurality of contacts (12) (small dashed-circles) consisting of stretchable, thin and flexible electrodes connected to a conductor. The electrode elements may be positioned on the border of the expandable assembly (3) as well as on the surface of said expandable assembly (1). The border of the expandable assembly (3) may have a polygonal shape (b) or a circular shape (c).

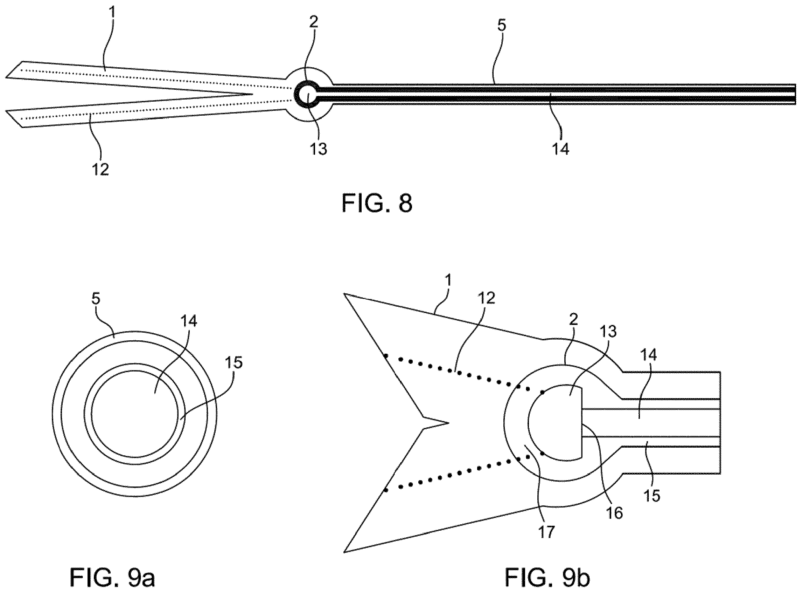

Figure 8 shows the guide wire (5) according to the third aspect of the present disclosure comprising an expandable assembly (1); one or a plurality of contacts (12) (dotted-line) positioned on said expandable assembly (1), wherein each contact (12) comprises an electrode element connected to a conductor (not shown); an electrically conducting pivotable joint (13) connecting said expandable assembly (1) to said guide wire (5), wherein said guide wire (5) has an insulated conductive core (14), and it is connected to a power supply (not shown) capable of selectively generating an electrical signal transmitted to said one or a plurality of contacts (12) via conductive core (14) of said guide wire (5).
Figure 9 shows in a) a section of the guide wire (5) according to the third aspect of the present disclosure, wherein said guide wire (5) has a conductive core (14) and an insulating material (15) is provided between said conductive core (14) and the inner wall of the guide wire (5); b) the pivotable joint (2) connecting the expandable assembly (1) to said guide wire (5) has to be electrically conductive. In this respect, the pivotable joint (2) of the present disclosure comprises: a spherical member of electrically conducting material (13) having an aperture (16) for the receipt of the conductive core (14) of said guide wire (5), electrical connection to one or a plurality of contacts (12) of the expandable assembly (1) and a housing of electrically insulating material (17) and means supporting the electrical connecting means in said housing (not shown).

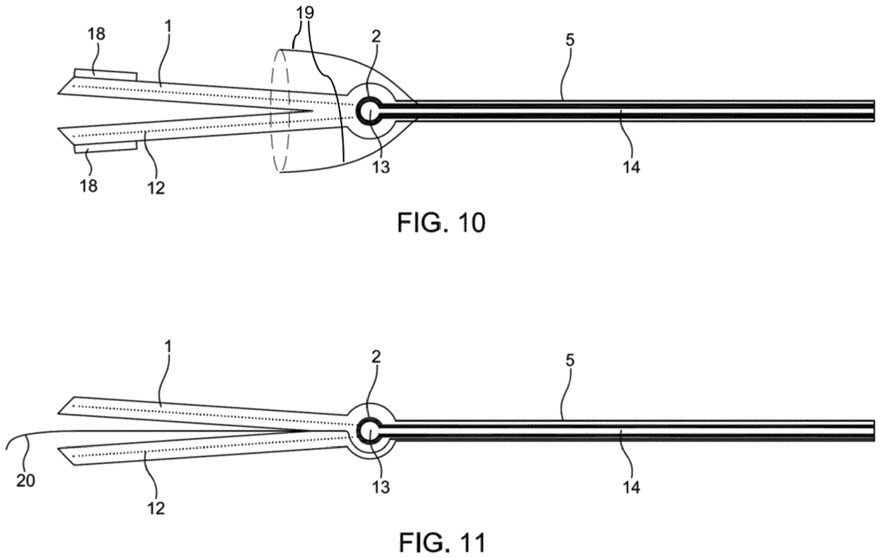

Figure 10 shows the guide wire (5) according to the third aspect of the present disclosure. In particular, a preferred location of one or a plurality of contacts (12) is represented by the distal end (18) of said expandable assembly (1). Further contact locations may be conceived such as an additional electrically conducting wire(s) (19).
Figure 11 shows the guide wire (5) according to the third aspect of the present disclosure. In particular, an integrated electrically conductive guide wire (20) preferably having an angled shaped tip connected to a part of the device distal to the pivotable joint (2) may be used to produce vasospasm.

### Description of the invention

In several situations like blood vessel injuries or during surgical or endovascular treatments it may be necessary to block the blood flow. In this respect, there are different options including permanent implantable devices like Amplatzer for large vessels or coiled wire for small vessels. Filters are used for temporary prevention of particle embolization, e.g. during carotid stenting, but they do not stop the blood flow. For temporary and fully reversible vessel closure, just a few devices have been described, all using balloons. Balloons are well applicable in rather straight vessels, where they provide a closure pattern rectangular to the vessel axis. In other situations of temporary vessel occlusion, like during application of sclerosants for the treatment of vein segments, it is required a very precise discrimination of diseased and healthy segments or branches, which may not be achievable with balloons. Furthermore, balloons have to be inflated with pressure to tighten which may induce damages to the vessel. Thus, there is no device to provide a precise and temporary occlusion in tortuous or branched segments or junctions which are not rectangular such as the majority of blood vessels. A solution to this problem is provided by the present disclosure relating to a new guide wire for use in a catheter assembly, comprising an expandable assembly and a pivotable joint that connects the expandable assembly to a steerable guide wire.

It will be appreciated by the person skilled in the art that the device according to the present disclosure provides significant advantages over the current occluding devices. The expandable assembly of the steerable guide wire advantageously provides temporary occlusion of the vessel due to an adhesive mechanism for self-anchoring. Thus, the self-anchoring of the expanded assembly completely eliminates the problem of migration of the permanent occluding devices or parts of it and subsequent issues associated with said phenomena.

Further advantages include ease of positioning and efficiency in occluding a venous vessel segment due to its repositionability; since it may be retrieved, it may be easily repositioned, providing thus, a prompt correction of its misplacement. Yet another advantage of the present device is represented by the presence of a pivotable joint aimed to occlude precisely the target vessel segment in tortuous or branched vasculature. A further advantage of the device disclosed herein is the presence of a steerable guide wire that facilitates navigation through the patient's tortuous vessels.

According to a first aspect of the present disclosure, disclosed herein is a guide wire (5) for use in a catheter assembly comprising: a distal end expandable assembly (1) and a pivotable joint (2) connecting said expandable assembly (1) to said guide wire (5) (Fig. 1). As used herein, the term "catheter assembly" refers to a device that can be inserted into a human body and used to temporary occlude a target vessel segment in said body. In the context of the present invention the term "vessel" refers to any tubular cavity within the body of a subject. As used herein, the term "vessel" comprises, for example, blood vessels, such as arteries, veins and capillaries. Preferred vessels are characterized by non-elastic wall, e.g. veins. As used herein, the term "expandable" generally refers to the ability of a material and/or structure to increase in size and/or volume.

Further, in the context of the present disclosure the term "pivotable" refers to mechanisms that provide pivoting motion.

It is worth noting that the position of the pivotable joint is not necessarily at the basis of the expandable part. There may be, for reasons of vessel anatomy, a linear or curved link between the basis of the expandable assembly and the pivotable joint.

According to a first aspect of the present disclosure, the guide wire (5) of the present disclosure is steerable. Thus, it is designed to navigate vessels and reach the target segment within a tortuous and/or branched vessel segment. As used herein, the term "steerable" relates to the ability and responsiveness of the guide wire (5) to navigate vessels. In order to ensure these features, the guide wire (5) of the present disclosure is further characterized by pushability and torque. Pushability is defined as the amount of force needed to advance the wire. Torque is the response of the wire to turning by the operator when navigating vessels. In the context of the present disclosure, the guide wire (5) may have different structures, i.e. solid steel or nitinol core wire, solid core wire wrapped in a smaller wire coil or braid. Coiled or braided wire offers a large amount of flexibility, pushability and twist resistance. Nitinol wire, used by itself or braided with stainless steel, helps increase flexibility and allows the wire to spring back into shape after navigating a tortuous vessel segment. According to the present disclosure, the guide wire (5) may be coated with a polymer, such as silicone or polytetrafluoroethylene (PTFE), to increase its lubricity. The guide wire (5) may also include a hydrophilic coating to reduce friction during deployment and for easier movement in tortuous vessels.

According to the first aspect of the present disclosure, the expandable assembly (1) is capable of being changed from a retracted state to an expanded state (Fig. 2). In the context of the present disclosure, the expandable assembly (1) is arranged in a substantially conical configuration. As used herein, the term "substantially" refers to the ability of the expandable assembly (1) to exhibit sufficient features for being associated to a conical shape. Thus, said expandable assembly (1), in its expanded state, may be of any suitable configuration including: bowl-shaped, mushroom-shaped, cup-shaped, helical-shaped, disc-shaped, bulbous-shaped, calyx-shaped, umbrella-shaped or any combination of the above. The preferred geometry of the expandable assembly (1) in its expanded state is intended to adhere correctly to the lumen wall of the target vessel segment or to temporarily occlude the opening of the target vessel from the side of the larger adjacent healthy vessel. Further, the border of said the expandable assembly (3) may have a polygonal shape or a circular shape (Fig. 2 b,c). As used herein, the term "polygonal" refers to the shape of a plane geometric polygon, optionally with rounded corners, having three or more vertices.

Further configurations of said expandable assembly (1) may be conceived according to the present disclosure.

For example, the expandable assembly (1) may be a clamp-like device, wherein the two arms of the clamp are connected by a connecting element, e.g. in the shape of a spring, to support the folding and/or the unfolding of the expandable assembly (1). In this case, the device disclosed herein may consist of a single wire loop or several parts interconnected. According to this configuration of the expandable assembly (1), the guide wire may be also included to form one single wire which is bent in a particular way. In this respect, the joint may be represented by a movable link, e.g. a nut, or a floppy section of the wire.

According to another embodiment of the present disclosure, the expandable assembly (1) of the guide wire (5) is retrievable and resiliently deformable in a retrievable configuration. As used herein, the term "resiliently deformable" relates to the ability of the expandable assembly (1) of the guide wire (5) to change from a first shape to a second shape and to return to the first shape. In the context of the present disclosure, the expandable assembly (1) may change its configuration in relation to its exposure from the catheter assembly. Consequently, the expandable assembly (1) may be made of any shapeable material or a shape memory material.

According to another embodiment, the expandable assembly (1) comprises an impermeable membrane, wherein said membrane is made of a shape memory material and has a net-like filter structure. Thus, the expandable assembly (1) is made by any suitable material aiming to block the blood flow. In the context of the present disclosure, suitable materials comprise elastic and non-elastic impermeable membranes. As used herein the term "impermeable" means 100% impermeable to blood. According to another embodiment, the expandable assembly (1) comprises a net-like filter structure. This feature allows the destruction of gas bubbles that may occur while performing sclerotherapy into the target vessel. These features of the expandable assembly (1) are of particular relevance in order to ensure an efficient temporary occlusion of the target vessel and a correct function of the expandable assembly (1) in both retracted and expanded state.

According to another embodiment of the present disclosure, the expandable assembly (1) of the guide wire (5) has an expansion ratio ranging from at least 1:5 to at least 1:10 of its diameter before and after expansion. As used herein, the term "at least" means "greater than" or "equal to" a referred value.

According to another embodiment, the expandable assembly (1) is configured to adhere the inner wall of a vessel lumen by means of at least one adhesive component (4), when said expandable assembly (1) is in its expanded state. In the context of the present application, an adhesive component (4) can be an adhesive, glue or any other bonding or sealing agent suitable for achieving a temporary bond between the border of the expanded assembly (3) and the internal wall of the vessel intended to occlude. Suitable bonding agents or adhesives may include, for example, collagen gels, pastes, gelatin and polysaccharide- (chitosan, alginate, heparin or chondroitin sulfate) based adhesives or other agents including reactive monomers or polymers, or any other elastomeric biocompatible material. The terms "bonding agent" and "adhesive" can be used interchangeably and refer to polymeric compositions useful to temporarily favors adhesion or sealing of the expandable assembly (3) and the vessel lumen. Suitable bonding agents or adhesives are advantageous since they ensure a time-limited function and are subjected to biodegradation *in vivo* after being contacted by a sclerosing agent, e.g. ScleroGlue^{®}, thus avoiding sclerosant foam migration within the target vessel segment.

According to another embodiment of the present disclosure, temporary adhesive component may comprise shape-changing materials, such as electro-active polymers (EAP). EAP materials include carbon nanotubes, conductive polymers, ionic polymer gels and ionic polymer metal composites. Ionic polymers are a class of active material that exhibit large bending deflections under the application of an electric field. The bending configuration of EAP persists temporarily and after that the material is subjected to a relax state and back to the initial configuration. Therefore, said limited bending configuration of EAP may be suitable for ensuring a temporary vessel occlusion. In the context of the present disclosure, the expandable assembly (1) in its expanded state is conceived to ensure a temporary occlusion of the blood vessel lumen. As reported herein, the term "occlusion" refers to a condition wherein the 100% of the blood vessel lumen is occluded. As used herein, the term "blood vessel" refers to a venous blood vessel.

According to another embodiment, the pivotable joint (2) of the guide wire (5) of the present disclosure allows for deflecting of the expandable assembly (1) in two or three dimensions relative to said guide wire (Fig. 3). In the context of the present disclosure, said pivotable joint (2) may allow different movements of the expandable assembly 1) with reference to its guide wire (5) comprising:
rotation, protraction, retraction, flexion, adduction and abduction. In particular, the pivotable joint (2) as disclosed herein allows a range of movement of expandable assembly (1) with reference to its guide wire (5) from 0 to 180 degree, preferably from 0 to 90 degree, more preferably from 0 to 60 degree.

According to another embodiment, the pivotable joint (2) is selected from the group consisting of: non-axial joint, uniaxial joint, biaxial joint, multiaxial joint. This feature ensures the precise and correct positioning of the expanded assembly (1) in tortuous and/or branched vessel segments.

An appropriate control device can be envisioned for governing the steerable guide wire of the present disclosure and its expandable assembly.

According to a second aspect, forming part of the invention, disclosed herein is a catheter assembly comprising a) a first tube (6), b) a guide wire (5) according to the first aspect and its relative embodiments, located within said first tube (6), c) optionally wherein said guide wire (5) is located in a second tube (7)positioned within said first tube (6) (Fig. 4). As used herein, the term "optionally" refers to the possibility that an event or circumstance may or may not occur. For example, in the context of the present application, said catheter assembly may or may not include a second tube (7) comprising the guide wire (5) disclosed in the present invention.

A catheter is a medically used tube for the transportation of fluids, gels, foams, pulps or gases. A tube is defined as a cylindrical hollow body with a length much larger than its diameter. In the context of the present invention, the relation of length to diameter is at least 50. The injection-evacuation catheter device according to the present invention may comprise one or two plastic tubes, wherein a first plastic tube (6) contains a second plastic tube (7) (Fig. 4). In case of a double-tube configuration, said tubes are spaced apart to provide room for the injection or evacuation of larger quantities of substances via the first tube (6). All tubes have openings at both ends and all are fully relocatable and demountable (Fig. 4). As used herein, the term "plastic" means a material of a wide range of synthetic or semi-synthetic organic solids that are mouldable. Plastics are typically organic polymers of high molecular mass, but they often contain other substances. They are usually synthetic, most commonly derived from petrochemicals, but many are partially natural. The length of the catheter according to the present invention may be 10-120 cm. The length of the guide wire (5) may correspond to 200-240% of the catheter length. The material of the tubes should be smooth, flexible and non-sticking.

According to the second aspect of the present invention, the first (6) and/or the second tube (7) is made of or covered with non-sticking material, comprising polytetrafluoroethylene (PTFE) or perfluoroalkoxy (PFA).

According to the second aspect of the present invention, the catheter assembly comprises one or more side holes (8) for injection and/or aspiration of fluids. When the catheter is characterized by side holes (8), a fluid may be injected and/or evacuated while the expandable assembly (1) is in retracted state and positioned at distal end (10) within said catheter. In the context of the present invention, the injection and/or aspiration of a fluid may occur before or after the release of the expandable assembly (1) connected to a guide wire (5) from the catheter. To fulfil these purposes in different anatomic situations, in a preferred embodiment there is at least one hole (8) provided in the wall of the first tube (6), but more holes (8) may be an option (Fig. 4). A single hole (8) is located at a position defined by 15-25 times the inner catheter diameter. Multiple side holes (8) are beginning in the same position from the distal end (10) of the catheter. The size of a single side hole (8) is preferably equal to 80-100% of the catheter's inner lumen, or, in case of multiple side holes (8), each varying between 60 and 80% of the catheter's inner lumen. As disclosed herein, the term "lumen" refers to the free inner diameter. It defines the space available within the tube. The side holes (8) may be different in size, shape and distribution. For example, the most proximal side hole may be larger than the others. The inventor found that the optimal size, shape and distribution of side holes may depend on the agents to be administered, for example sclerosant foams of high viscosity may require larger side holes. A foam deployment for sclerotherapy is regarded as optimal if the substance spreads uniformly around the tool, and keeps a uniform and distinct front line during injection to achieve precise placement. The optimal ratio for precise sclerosant foam treatment is met when the catheter volume along a foam quantity is equal to the volume of deployed foam. In this case, withdrawal of the catheter will provide exactly the space required to take the total foam quantity when the drug-triggered spasm contracts the vein. Hence, foam will not spread above the target zone. Furthermore, the volume of the catheter will reduce the quantity of sclerosant foam required to fill the vessel.

In case of a double-tube configuration (Fig. 4), the tubes work together as a functional unit: the second tube (7) is pre-mounted to contain the expandable parts in contracted position, limiting their outer diameter and facilitating introduction and advancement through the target vein. The first tube (6) offers a larger diameter for adequate sclerosant administration.

According to one embodiment of the second aspect of the present invention, a third tube may also be encompassed by the present catheter assembly. Said third tube is located outside the first tube (6) and fulfils the task of covering or uncovering the side hole(s) (8) of the first tube (6) for an optimal foam administration. For example, in one position, all side holes (8) are covered. In another position, one side hole (8) is opened. In another position, more or all side holes (8) are opened. The second tube (7) may have a grip or handle at the proximal end (11) for easier movements. There also may be markings or other signals or signal devices at or within the tubes to determine the status of the outer tubes' side holes, while the system is positioned within the patient's body.

According to another embodiment, the first tube (6) of the catheter device has an inner diameter ranging from 0.6 to 2.2 mm an outer diameter ranging from 0.8 to 2.8 mm and a length of 15 to 85 cm. A used herein, the term "outer diameter" refers to the diameter of the outer tube of the catheter assembly including the wall thickness. The term "inner diameter" refers to the diameter of the inner tube of the catheter assembly excluding the wall thickness. The shape of the catheter may be straight, or slightly bent (5-20 degrees) to steer along curved vessels or navigate through junctions. For deployment of substances, the first tube (6) may have one or several side holes to achieve uniform placement of the substance along a segment to treat. If the device includes a second tube (7) this may have a tempered tip for easier introduction. The second tube (7) may also, alone or with a guide wire support, serve as an introduction aid like a stiff guide wire. If introduced to a target vein as the first part, larger parts of the catheter system may be advanced by pushing them along the second tube (7). It may furthermore serve to deploy fluid agents and/or rinsing sclerosant, or serve as an outlet valve during injection or evacuation actions of the first tube (6). Said first tube (6) may serve to: 1) evacuate blood from the target vessel before application of sclerosant agent or the application of thermo-occluding techniques; 2) maintain evacuation and/or negative pressure during these applications; 3) administer saline or other liquids for rinsing if required by the used technique; 4) administer sclerosant foam; and 5) evacuate said sclerosant foam.

According to the invention, the catheter assembly comprises a sliding sleeve (9) which accommodates the expandable assembly (1) in a retracted state (Figs. 4, 5). The sliding sleeve (9) has a double function: it allows storing and delivering correctly the expandable assembly (1) within the catheter (from the proximal end (11) to distal end (10), and vice versa) and prevents the accidental opening of the expandable assembly (1) within said catheter. In the context of the present invention the sliding sleeve (9) refers to a short and tapered thin-walled cylinder just for the purpose of introduction of the expandable assembly into the catheter or a long cylinder comprised within the first tube (6) of the catheter device, having a length similar to the first tube (6) and intended to cover the expandable assembly (1) or to keep said expandable assembly (1) compressed until its delivery. Said sliding sleeve (9) may be made from polymer film of different thickness, e.g. comprising polytetrafluoroethylene (PTFE), perfluoroalkoxy (PFA) or fluorinated ethylene propylene (FEP). As used herein, the term "proximal end" refers to a location on the catheter assembly of this invention that is closest to the clinician using the device and farthest from the patient in connection with whom the device is used. The term "distal end" refers to a location on the catheter assembly of this invention that is farthest from the clinician using the device and closest to the patient in connection with whom the device is used. In the context of the present invention, the term "patient" refers to a subject in need of a treatment based on the use of a steerable guide wire and/or a catheter assembly according to the present invention.

According to another embodiment, the catheter assembly of the present invention is conceived to temporary occlude tortuous and/or branched vessel segments in mammals. This function can be carried out by the guide wire of the present invention. As used herein, the term "mammals" refers, but not limited, to human subjects including any subject in need of the device disclosed herein, preferably adult and/or elderly subjects.

According to another embodiment of the present invention, the catheter assembly is suitable for use in the treatment of venous diseases. As used herein, the term "venous diseases" refers to an altered physiological state of the internal walls of a leg vein segment of a subject, wherein the vein valves of that segment are deteriorated, defective and/or incompetent. In such altered state, either in the deep or superficial vein system, blood flows backwards towards the foot of a subject determining several complications. Resulting from a blood clot or an inherited abnormality of the vein wall, venous diseases can be classified in superficial (varicose veins) and deep (chronic venous insufficiency). In the context of the present invention, a preferred vein segment target is represented by superficial veins.

According to another embodiment of the present disclosure, the use of the catheter assembly comprises the following steps (all steps may use ultrasound monitoring) (Fig. 6):
a. Vein access by hollow needle puncture;
b. Insertion of a standard guide wire;
c. Advancing the catheter assembly(e.g. PhleboCath, with side holes) to the desired position;
d. Removal of the standard guide wire;
e. Loading of the guide wire (5) into the catheter, wherein the expandable assembly (1) is temporarily locked in a retracted state within a sliding sleeve (9) of said catheter;
f. Advancing the expandable assembly (1) within the catheter by pushing the attached guide wire (5);
g. Release of the expandable assembly (1) from the sliding sleeve (9) either when inside of the catheter or when reaching its distal position within the catheter;
h. Positioning the catheter assembly over the location intended to occlude;
i. Withdrawing the catheter while the guide wire (5) is kept in place, until the expandable assembly (1) is released and expanded;
j. Optionally, electric current via one or a plurality of contacts (12) may be used to retract the vein walls to support tight occlusion and/or reduce the target vein diameter;
k. Retracting and positioning of the expanded assembly (1) by pulling the guide wire (5) to ensure the occlusion of the target vessel;
l. Optionally, testing closure by injecting air-foamed saline or ultrasound contrast medium;
m. Performing foam sclerotherapy: positioning of the catheter according to the needs of sclerotherapy, aspiration of remaining blood or elevation of leg to empty blood load, injection of sclerosant foam, dwelling time 1-3 minutes, optionally: Aspiration of foam. Foaming (i.e. sclerosant administration) may be performed in different ways through several side holes (8) or via one front hole or side hole during retraction of the catheter;
n. Advancing the catheter to collect the expanded assembly (1);
o. Retracting the expanded assembly (1) by pulling its guide wire (5) to a position within the catheter;
p. Optionally further sclerofoam deployments may be performed, e.g. to fill side branches or perforator veins, during catheter withdrawal.
q. Optionally the occlusion device may be used in one or several further locations, depending on the requirements of sclerotherapy, e.g. to protect healthy veins;
r. Removal of the guide wire (5) and the catheter from the vein.

As used herein, the term "foam sclerotherapy" refers to a composition comprising a sclerosant agent and having substance formed by trapping pockets of gas in liquid. In the context of the present disclosure, alternative pharmaceutical formulation may be encompassed comprising the sclerosant agent, such as gel or liquid.

In another embodiment, a hemostatic sheath may be installed in Seldinger technique, serving as access for the occluding device and the catheter/catheters. When using the temporary occluding device during a vein gluing procedure, the access may be from a non-target part of the venous system to avoid interference of catheters, wires and glue.

While the preferred embodiment relates to protect healthy vein vessels from sclerosant foam placed in diseased veins, the present disclosure may comprise other applications. For example, blood vessels to be treated may be discriminated from healthy ones according to their respective mechanical properties such as elasticity. In this respect, the elasticity of said blood vessel may be tested according to different method known in the art, for example by using electrical stimulation. Said electrical stimulation may be induced by one or a plurality of contacts (12) positioned on the expandable assembly (1) of the guide wire (5) of the present disclosure.

The different response of the blood vessel segment to such electrical stimulus of different intensities may discriminate damaged or diseased blood vessels from healthy ones.

A third aspect of the present disclosure relates to a guide wire (5) for use in a catheter assembly comprising: an expandable assembly (1) according to the first aspect of the disclosure; one or a plurality of contacts (12) positioned on said expandable assembly (1), wherein each contact comprises an electrode element connected to a conductor; an electrically conducting pivotable joint (2) connecting said expandable assembly (1) to said guide wire (5), wherein said guide wire (5) has an insulated conductive core (14), and a power supply capable of selectively generating an electrical signal transmitted to said plurality of contacts (12) via conductive core (14) of said guide wire (5) (Figs. 7-9). As used herein, the term "electrode element" refers to one or more conductive elements formed from any suitable metallic material, such as stainless steel, nickel, titanium, tungsten, and the like, connected via a conductor to the conductive core (14) disposed within the guide wire (5), and thus, to a power supply capable of selectively generating an electrical signal. The transmission of electrical impulses is ensured by an electrically conductive pivotable joint (14). In particular, said pivotable joint comprises: a spherical element (13) of electrically conducting material having an aperture (16) for receiving the conductive core (14) of said guide wire (5) and electrical connection to the conductor of the expandable assembly (1), a housing of electrically insulating material (17) and means supporting the electrical connecting means in said housing. As used herein, the term "electrically conducting material" refers to a material that is a conductor of electricity, which includes, for example, but is not limited to pure metal or alloys. As used herein, the term "plurality" refers to any integer greater than 1. Thus, the expandable assembly disclosed herein may be characterized by one or more contacts in order to achieve a selective and suitable vasospasm of the target vessel segment. In the context of the present disclosure, the stimulation by means of the contacts (12) may be monopolar, bi-polar or multi-polar.

The inventor has found that the combination of using an electrical inductive element with the catheter according to the present invention would be advantageous in determining a temporary and controlled modification of the blood vessel lumen (vasospasm).

According to the third aspect of the present disclosure, the guide wire is characterized by an insulated conductive core (14) made of an electrically conductive material and has an essentially constant diameter over its entire length. An insulating material (15) is provided between said conductive core (14) and said inner wall of the guide wire (5).

According to the third aspect of the present disclosure, the guide wire (5) is connected to a power supply capable of selectively generating an electrical signal transmitted to said plurality of contacts disposed on the expandable assembly. The power supply includes a power source such as a battery for connecting to external power, a circuit for controlling the waveform characteristics of the electrical impulse, and means for sending the electrical impulse through the electrodes and suitable controls. The circuit for controlling the waveform characteristics may include means for customizing the frequency, voltage, length of pulses and number of pulses, or overall duration or means for selecting preloaded waveform characteristic profiles.

According to another embodiment of the third aspect of the present disclosure, the electrode elements have to be thin, flexible and stretchable so they can adapt to the contours and/or surface of the expandable assembly (1) to which they are attached.

According to another embodiment of the third aspect of the present disclosure, the activation of said one or a plurality of contacts (12) produces a vasospasm of the vessel lumen of the target vein.

The purpose of the electrodes is to apply electric current to the vein wall to achieve just as much contraction as required to tightly adapt the vein wall to the temporary occlusion device. Generally, a vasospasm may be defined as a prolonged constriction of a blood vessel lumen resulting from local vascular hyperactivity to a vasoconstrictive stimulus. Said constriction reduces blood flow to the area of the body served by the blood vessel and if maintained for a sufficient length of time, results in symptoms of ischemia, such as pain, loss of function, or cell death. As used herein, the term "vasospasm" is referred to a venous vessel lumen constriction that persists for at least 5 minutes.

In the context of the present disclosure, a preferred location of one or a plurality of contacts (12) is represented by the distal end (18) of said expandable assembly (1). Further contact locations may be conceived; for example, additional flexible wire(s) (19) could also be used (Fig. 10). Said flexible wire(s) (19) has to be electrically conductive. As a further option, an integrated guide wire (20) may be used (Fig. 11).

In the context of the present disclosure, the shape of the integrated guide wire (20) exceeding the expandable assembly may be of any shape. However, J-shaped may be preferred because of better steering.

According to another embodiment of the third aspect of the present application, one or a plurality of contacts (12) is activated when said expandable assembly (1) is in the expanded state. This feature ensures a safe and precise action of the expandable assembly (1) to reduce the vessel lumen.

Pulsed electrical stimulation can induce reversible vasoconstriction and permanent occlusion in veins. Suitable set of values for the electric signal to determine temporary vasoconstriction are known in the art (Bioelectromagnetics 2008, 29 (2): 100-107).

## Claims

1. A catheter assembly for temporary blood vessel occlusion comprising:
a. a steerable guide wire (5)
b. an expandable assembly (1),
c. a pivotable joint (2), wherein said pivotable joint connects said expandable assembly (1) to said guide wire (5),
wherein said expandable assembly (1) is capable of being changed from a retracted state to an expanded state and wherein said expandable assembly (1) is arranged in a substantially conical configuration;
wherein said catheter assembly further comprising a first tube (6), wherein the guide wire (5) is located within said first tube (6),
wherein said first tube (6) is made of or covered with a non-sticking material comprising polytetrafluoroethylene (PTFE) or perfluoroalkoxy (PFA) and, wherein said first tube (7) comprises one or more side holes (8) configured for injection and/or aspiration of fluids,
wherein said catheter assembly comprises a sliding sleeve (9) accommodating the expandable assembly (1) in a retracted state within said catheter assembly.

2. The catheter assembly according to claim 1, wherein said expandable assembly (1) is retrievable and resiliently deformable in a retrievable configuration.

3. The catheter assembly according to any of the claims 1 and 2, wherein said expandable assembly (1) comprises an impermeable membrane, wherein said membrane is made of a shape memory material and has a net-like filter structure.

4. The catheter assembly according to the claims 1 to 3, wherein said expandable assembly (1) has an expansion ratio ranging from at least 1:5 to at least 1:10 of its diameter before and after expansion.

5. The catheter assembly according to any of the claims 1 to 4, wherein said expandable assembly (1) is configured to adhere to the inner wall of a vessel lumen by means of at least one adhesive component (4).

6. The catheter assembly according to any of the claims 1 to 5, wherein the pivotable joint (2) of said guide wire (5) allows for deflecting the expandable assembly (1) in two or three dimensions relative to said guide wire (5).

7. The catheter assembly according to claim 6, wherein said pivotable joint (2) is selected from the group consisting of: non-axial joint, uniaxial joint, biaxial joint, multiaxial joint.

8. The catheter assembly according to any of the claims 1 to 7, wherein said first tube (6) has an inner diameter ranging from 0.6 to 2.2 mm, an outer diameter ranging from 0.8 to 2.8 mm and a length of 15 to 85 cm.

9. The catheter assembly according to any of the claims 1 to 8, wherein said catheter assembly is conceived to temporary occlude tortuous and/or branched vessel segments in mammals.

## Patentansprüche

1. Eine Katheterbaugruppe für den vorübergehenden Verschluss von Blutgefäßen, umfassend:
a. ein lenkbarer Führungsdraht (5)
b. eine ausziehbare Baugruppe (1),
c. ein drehbares Gelenk (2), wobei das drehbare Gelenk die ausziehbare Baugruppe (1) mit dem Führungsdraht (5) verbindet,
wobei die ausziehbare Baugruppe (1) imstande ist von einem eingezogenen Zustand in einen ausgezogenen Zustand umgewandelt zu werden und wobei die ausziehbare Baugruppe (1) in einer im Wesentlichen konischen Konfiguration angeordnet ist;
wobei die Katheterbaugruppe ferner umfasst
einen ersten Schlauch (6), wobei sich der Führungsdraht (5) innerhalb des ersten Schlauches (6) befindet,
wobei der erste Schlauch (6) aus einem nicht-haftenden Material gemacht ist oder mit einem nicht-haftenden Material überzogen ist, das Polytetrafluorethylen (PTFE) oder Perfluoralkoxy (PFA) umfasst und, wobei der erste Schlauch (7) ein oder mehrere Seitenlöcher (8) umfasst, die für die Injektion und/oder das Absaugen von Flüssigkeiten konfiguriert sind,
wobei die Katheterbaugruppe eine Schiebehülse (9) umfasst, die die ausziehbare Baugruppe (1) in einem eingezogenen Zustand innerhalb der Katheterbaugruppe beherbergt.

2. Die Katheterbaugruppe nach Anspruch 1, wobei die ausziehbare Baugruppe (1) zurückziehbar ist und in einer zurückgezogenen Konfiguration elastisch deformierbar ist.

3. Die Katheterbaugruppe nach einem der Ansprüche 1 und 2, wobei die ausziehbare Baugruppe (1) eine undurchlässige Membran umfasst, wobei die Membran aus einem Formgedächtnismaterial gemacht ist und eine netzartige Filterstruktur aufweist.

4. Die Katheterbaugruppe nach den Ansprüchen 1 bis 3, wobei die ausziehbare Baugruppe (1) ein Expansionsverhältnis von mindestens 1:5 bis mindestens 1:10 ihres Durchmessers vor und nach dem Ausziehen aufweist.

5. Die Katheterbaugruppe nach einem der Ansprüche 1 bis 4, wobei die ausziehbare Baugruppe (1) so konfiguriert ist, dass sie mittels mindestens einer Klebekomponente (4) an der Innenwand eines Gefäßlumens haftet.

6. Die Katheterbaugruppe nach einem der Ansprüche 1 bis 5, wobei das drehbare Gelenk (2) des Führungsdrahtes (5) eine Ablenkung der ausziehbaren Baugruppe (1) in zwei oder drei Dimensionen relativ zum Führungsdraht (5) ermöglicht.

7. Die Katheterbaugruppe nach Anspruch 6, wobei das drehbare Gelenk (2) ausgewählt ist aus der Gruppe bestehend aus: nicht-axiales Gelenk, einachsiges Gelenk, biaxiales Gelenk, mehrachsiges Gelenk.

8. Die Katheterbaugruppe nach einem der Ansprüche 1 bis 7, wobei der erste Schlauch (6) einen Innendurchmesser von 0,6 bis 2,2 mm, einen Außendurchmesser von 0,8 bis 2,8 mm und eine Länge von 15 bis 85 cm aufweist.

9. Die Katheterbaugruppe nach einem der Ansprüche 1 bis 8, wobei die Katheterbaugruppe konzipiert ist, um gewundene und/oder verzweigte Gefäßsegmente in Säugetieren vorübergehend zu verschließen.

## Revendications

1. Un ensemble de cathéter pour l'occlusion temporaire des vaisseaux sanguins comprenant:
a. un fil de guidage orientable (5)
b. an assemblage extensible (1),
c. une jointure pivotante (2), dans laquelle ladite jointure pivotante relie ledit ensemble extensible (1) audit fil de guidage (5),
dans lequel ledit assemblage extensible (1) est susceptible d'être changé d'un état rétracté à un état étendu et dans lequel ledit assemblage extensible (1) est disposé dans une configuration substantiellement conique;
dans lequel ledit ensemble cathéter comprenant en outre
un premier tube (6), dans lequel le fil guide (5) est situé à l'intérieur dudit premier tube (6),
dans lequel ledit premier tube (6) est constitué ou recouvert d'un matériau antiadhésif comprenant du polytétrafluoroéthylène (PTFE) ou du perfluoroalcoxy (PFA) et, dans lequel ledit premier tube (7) comprend un ou plusieurs trous latéraux (8) configurés pour l'injection et/ou l'aspiration de fluides,
dans lequel ledit ensemble de cathéter comprend un manchon coulissant (9) logeant l'ensemble extensible (1) dans un état rétracté à l'intérieur dudit ensemble de cathéter.

2. L' ensemble de cathéter selon la revendication 1, dans lequel ledit ensemble extensible (1) est récupérable et élastiquement déformable dans une configuration récupérable.

3. L'ensemble de cathéter selon l'une quelconque des revendications 1 et 2, dans lequel ledit ensemble extensible (1) comprend une membrane imperméable, dans laquelle ladite membrane est constituée d'un matériau à mémoire de forme et a une structure filtrante en forme de filet.

4. L'ensemble de cathéter selon les revendications 1 à 3, dans lequel ledit ensemble extensible (1) a un rapport de dilatation allant d'au moins 1:5 à au moins 1:10 de son diamètre avant et après expansion.

5. L'ensemble de cathéter selon l'une quelconque des revendications 1 à 4, dans lequel ledit ensemble extensible (1) est configuré pour adhérer à la paroi interne d'un vaisseau lumineux au moyen d'au moins un composant adhésif (4).

6. L'ensemble de cathéter selon l'une quelconque des revendications 1 à 5, dans lequel la jointure pivotante (2) dudit fil de guidage (5) permet de dévier l'ensemble extensible (1) en deux ou trois dimensions par rapport audit fil de guidage (5).

7. L'ensemble de cathéter selon la revendication 6, dans lequel ladite jointure pivotante (2) est choisi dans le groupe constitué par : jointure non axiale, jointure uniaxiale, jointure biaxiale, jointure multiaxiale.

8. L'ensemble de cathéter selon l'une quelconque des revendications 1 à 7, dans lequel ledit premier tube (6) a un diamètre intérieur allant de 0,6 à 2,2 mm, un diamètre extérieur allant de 0,8 à 2,8 mm et une longueur de 15 à 85 cm.

9. L'ensemble de cathéter selon l'une quelconque des revendications 1 à 8, dans lequel ledit ensemble de cathéter est conçu pour obstruer temporairement des segments de vaisseaux tortueux et/ou ramifiés chez les mammifères.
